# EUROPEAN PATENT APPLICATION

(11) **EP 1 138 305 A1**
(43) Date of publication of application: **04.10.2001**
(21) Application number: 00306289.0
(22) Date of filing: 24.07.2000
(51) Int. Cl.: A61G 13/00, A61H 33/00

(54) **Apparatus for carrying out a waterbirth**

(30) Priority: 30.03.2000 KR 0016544
(71) Applicant: Yang, Won Dong, Gwanak-ku, Seoul (KR)
(72) Inventor: Yang, Won Dong, Gwanak-ku, Seoul (KR)
(74) Representative: Jacob, Reuben Ellis

(57) **Abstract**

Disclosed in a waterbirth apparatus (1) comprising a birth tub (10) into which purified water is supplied and in which a parturient woman delivers a baby in the water. The birth tub (10) stores the purified water and the parturient woman is received therein. A water purifying unit (40) purifies the water supplied from a water supplying unit (38) and supplies the purified water into the birth tub (10). With this configuration, the labor or pain of the parturient woman experienced in the course of delivery is relieved to some degree.

## Description

The present invention relates to apparatuses for carrying out waterbirth, and more particularly, to a waterbirth apparatus comprising a birth tub into which purified water including a physiological saline solution is supplied, and in which a parturient woman delivers a child in the water.

Generally, to reduce labor or pain of a parturient woman while giving birth to a child, a physical stimulus by an external force has been given to her or a parturifacient has been injected to her. Otherwise, a surgical operation such as Caesarean operation has been performed or a separate delivery apparatus has been used.

The separate delivery apparatus mentioned above is disclosed in Korean Patent Laid-Open Nos. 99-72549 and 99-73255.

Recently, waterbirth has been highlighted. However, there. is currently no separate apparatus for waterbirth wherein a parturient woman is secure against or free from infection, etc. Instead, waterbirth has been performed in a bathtub or in the ocean, etc. But, the water used therein is not free from danger such as bacteria, etc.

For such reasons, the value of waterbirth itself has been recognized, but there has been a little understanding on the importance of the water used in the course of delivery.

The present invention has been proposed to solve the above-described shortcomings of the conventional waterbirth apparatuses. Accordingly, an object of the present invention is to provide a waterbirth apparatus wherein purified water is used during delivery and a desired rest of the parturient woman or her protection from bacteria, etc. is considered.

In accordance with the present invention, the above and other objects can be accomplished by a provision of a waterbirth apparatus comprising a birth tub for storing purified water supplied to be used during parturition and allowing a parturient woman to enter thereinto for delivery, and a water purifying unit installed on a water supplying unit for supplying water into the birth tub, for purifying the water supplied from the water supplying unit and supplying the purified water to the birth tub.

The birth tub may further comprise a temperature maintaining unit for keeping the water supplied into the birth tub within the range of 35°C to 40°C, and preferably, at the same degree as that of the human body, and a water level controller for controlling the level of the water at the birth tub to be in a predetermined level.

Advantageously, a chair for a parturient woman, having a downward hole, is installed in the birth tub, allowing her to have a baby while sitting on the chair.

A pump for pumping up the water to be supplied to the water purifying unit and a pre-treating unit for pre-treating the water may be installed between the water supplying unit and the water purifying unit. Further, a heating unit for heating to very quickly increase the temperature of the water may be installed between the water purifying unit and the birth tub.

Preferably, the water purifying unit is comprised of a reverse osmotic filter and a carbon filter, etc. More specifically, the filter may include a hollow fiber membrane filter, a ceramic filter, an ultra filtration(UF) filter, an anion resin or a cation resin, etc., in addition to the reverse osmotic filter and the carbon filter described above. The water purifying unit may be installed on the outside portion of the birth tub, both of which are preferably in a single body.

With this configuration, the parturition can be made in the birth tub into which the purified water is supplied continuously while giving birth to a baby.

The above and other objects, features and other advantageous of the present invention will be more clearly understood from the following detailed description given by way of example taken in conjunction with the accompanying drawings, in which:
Fig. 1 is a perspective view of a waterbirth apparatus according to one embodiment of the present invention; and
Fig. 2 is a block diagram illustrating the waterbirth apparatus comprising a water purifying unit of Fig. 1.

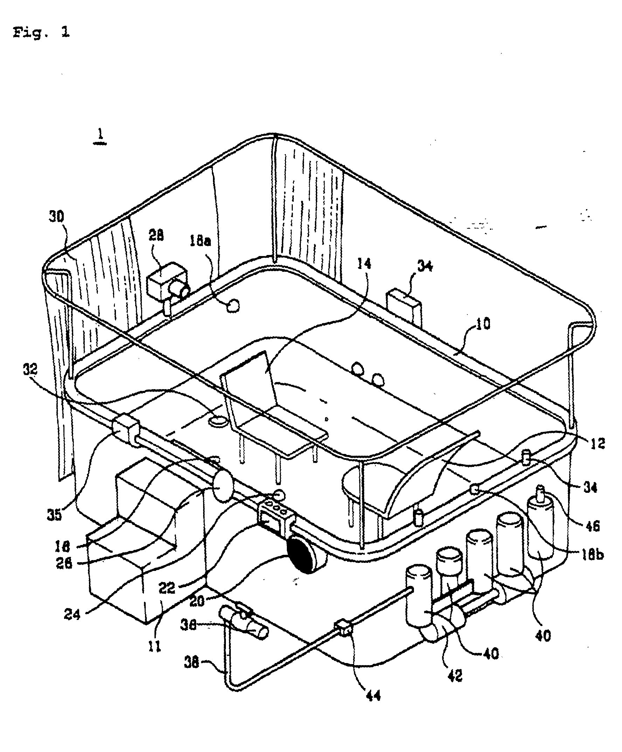

Referring to Fig. 1 showing a perspective view of a waterbirth apparatus according to one embodiment of the present invention, a waterbirth apparatus 1 is comprised of a birth tub 10 for storing purified water, the water being used in the course of waterbirth. The birth tub 10 is equipped with a chair 12 for a parturient woman, having a downward hole, for allowing the woman to have a baby with sitting thereon, and a chair 14 for her family or a midwife who looks after the parturient woman during the childbirth. The chair 12 for the parturient woman has elbow rests for allowing her to support herself and make herself comfortable. The chairs 12 and 14 can be fixed into the birth tub, or they can be removable so as to be adaptively moved according to the parturient woman and her attendant's poses. On the upper back of the chair 12 for the parturient woman is mounted a head rest, allowing her body to be rested comfortably thereon. The birth tub 10 can be made of fiberglass reinforced plastics(FRP), marble, stainless steel, polypropylene(PP), marble or ceramics, etc. and in the form of a planar square, a circle, a triangle, a diamond, etc.

The birth tub 10 is equipped with a temperature maintaining unit 16 for keeping the purified water within the range of 35°C to 40°C, and preferably, to the same degree as that of the human body, and water level controllers 18a and 18b for controlling the water in the birth tub to be in a predetermined level. The temperature maintaining unit 16 includes a cartridge heater, for maintaining the temperature of the purified water within the range of 35°C to 40°C, and preferably, to the same degree as that of the human body. The water level controllers have respectively a control sensor 18a and a control lamp 18b. The control sensor 18a controls supply of the water according to the result sensed by the water level control sensor 18a and the control lamp 18b indicates the level of the water supplied to the birth tub 10.

The birth tub 10 may be equipped with a bubbling member (not shown) for bubbling the water, and the bubbling member may include an air pump for circulating the supplied water to the birth tub 10. The birth tub 10 may also include an overload intercepting member (not shown) and electromagnetic wave intercepting member (not shown) for protecting the parturient woman from electric shock and electromagnetic waves.

The birth tub 10 may also include the following elements: a CD player 22 with a speaker 20, for allowing the parturient woman to listen to music, a lamp 24 for producing a variety of backgrounds according to the music, a perfume dispenser 26 for dispensing perfume, a camcorder 28 for taking film of the delivery of a baby and a curtain 30 for guarding the parturient woman's privacy from others, etc. To convert the water supplied into the birth tub 10 into a physiological saline solution, a salt container 35 for storing a medical salt may be installed to the birth tub 10.

The birth tub 10 includes a drain port 32 for draining out the used water, and a disinfectant container 34 for keeping a disinfectant to be used in disinfecting the birth tub 10 after parturition. The drain port 32 is used for promptly draining out the used water and the amniotic fluid introduced into the water during delivery, and the disinfectant kept in the disinfectant container 34 is used in disinfecting the birth tub 10. The birth tub 10 after draining and disinfecting can be continuously used for the next waterbirth. The draining port 32 is also used in draining off the water when the birth tub 10 is filled with water. The birth tub 10 also includes a water inlet 36 for supplying the water into the birth tub. The water inlet 36 is formed with an electronic solenoid valve. The valve intercepts supply of the water and prevents overflowing of the water when the birth tub 10 is filled with the water. The water inlet 36 is connected to those members that include a water purifying unit for supplying the water into the birth tub 10, which will be described later in detail.

The water purifying unit 40 is installed between the birth tub 10 and the water supplying unit 38 for supplying the water, for purifying the water supplied from the water supplying unit 38 and supplying the purified water into the birth tub 10.

The water purifying unit 40 includes a reverse osmotic filter and a carbon filter. More specifically, the filter includes a hollow fiber membrane filter, a ceramic filter, an ultra filtration(UF) filter, an anion resin or a cation resin, etc., in addition to the reverse osmotic filter and the carbon filter. The water purifying unit 40 can be configured to purify the water, making use of ozone, ultra violet rays, etc., with which a variety of foreign matters, heavy metals, ion or bacteria, etc. contained in the water supplied from the water supplying unit 38 can be purified through several steps. The water purifying unit 38 is comprised of a waste drain pipe for draining out the variety of foreign matters, heavy metals, ion or bacteria, etc. removed from the water. Where the birth tub 10 is not used for a long time and is to be kept, the birth tub 10 is covered with a vinyl cover for preventing pollution of the birth tub 10 due to contact with air, etc. The vinyl cover is not simply for keeping the birth tub, but for preventing airborne infection.

Between the water supplying unit 38 and the water purifying unit 40 are formed a pump for pumping the water to be supplied into the water purifying unit 40 and a pre-treating unit 44 for pre-treating the water. The pre-treating unit 44 is formed with a sediment filter including a filter paper or an unwoven fabric, etc., for preliminarily purifying the water. The pre-treating unit 44 is formed with a transparent case so that the time of replacing the filter or fabric is indicated outside. The pump 42 is further comprised of a pressure meter for measuring the pressure of water.

Between the water purifying unit 40 and the birth tub 10 is formed a heating unit 46 for very quickly increasing the temperature of the water. With this, the water supplied into the birth tub 10 can be maintained at an appropriate temperature. The temperature of the water after passing through the heating unit 46 is preferably 37°.

A water tank (not shown) for storing the water can be provided in the birth tub 10.

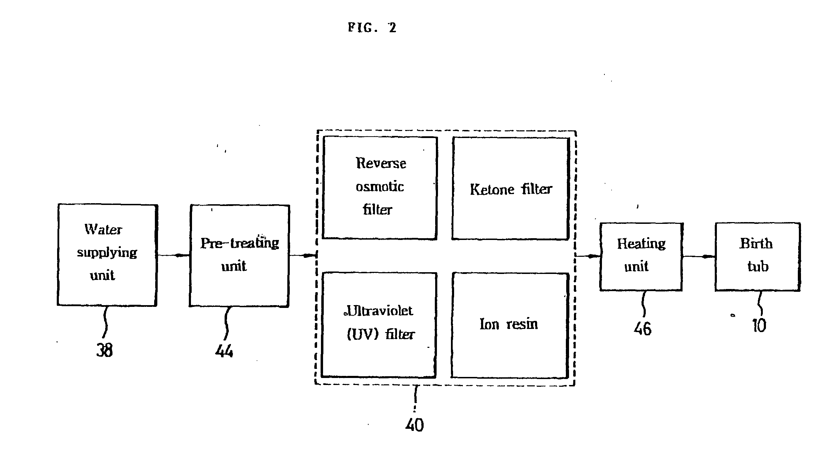

Fig. 2 is a block diagram illustrating a waterbirth apparatus including a water purifying unit. Referring to Fig. 2, a passage of the water supplied into the birth tub 10 from the water supplying unit 38 is depicted. First, the water is supplied from the water supplying unit 38. The water from the water source is used as the water therein. The water is preliminarily filtered, that is; purified while passing through the pre-treating unit 40. The water is finally purified while passing through a reverse osmotic filter, a carbon filter, an ultra filtration filter, and ion resins, etc. Subsequently, the water from the water purifying unit 40 is very quickly increased to around 37°C after passing through the heating unit 46, and this purified water is supplied into the birth tub.

The waterbirth apparatus according to the present invention is used in the following manner. First, a vinyl cover is removed from a birth tub and water is supplied into the birth tub. The water supplied into the tub is completely purified water through the above-described processes. A parturient woman is seated on the chair for her in the tub. Her family or a midwife may enter into the tub together. Using a CD player, a lamp, a perfume dispenser, etc., the atmosphere for childbirth is encouraged. The level and temperature of the water in the tub is continuously controlled during parturition. After she has a baby, the water contained in the tub, secretions, placenta, etc. are drained and discarded. While draining and discarding, the vinyl cover removed from the tub is properly disinfected and then discarded for prevention of infection. After this, the birth tub is ready for the next parturient woman. At the portion for draining is formed a sediment filter (carbon filter) for preventing environmental pollution due to discard of the secretions and placenta, etc. together with the water in the tub, and is further formed with a member such as a vacuum motor for forcibly draining out the water in. a short time.

As described above, the birth tub according to the present invention enables waterbirth to be performed in a secure and comfortable manner, making use of a tub for waterbirth, and supply members for supplying the purified water into the tub.

Because waterbirth is safely and comfortably performed, labor or pain of the parturient woman can be relieved to some degree and also the stress which the baby may experience during delivery can be reduced a bit. Because the parturient woman is seated on the chair, her pelvis is naturally extended, thereby making it easier to have a baby.

The bottom of the birth tub is embossed, to prevent the parturient woman from sliding. The steps installed inside and outside the birth tub are also helpful in her stepping in and out of the tub.

According to the present invention, an optimal environment for delivery is provided, by making use of a birth tub for safely and comfortably performing waterbirth, and supplying members for supplying the purified water into the birth tub.

Although a preferred embodiment of the present invention has been described for illustrative purposes, those skilled in the art will appreciate that various modifications, additions and substitutions are possible, without departing from the scope of the invention as disclosed in the accompanying claims.

## Claims

1. A waterbirth apparatus comprising:
a birth tub for storing purified water supplied to be used for waterbirth and allowing a parturient woman to enter thereinto for delivery; and
a water purifying unit installed between the birth tub and a water supplying unit for supplying water into the birth tub, said water purifying unit being used for purifying the water supplied from the water supplying unit and supplying the purified water to the birth tub.

2. The waterbirth apparatus according to claim 1, wherein said water purifying unit is installed in the outside portion of the birth tub in a single body.

3. The waterbirth apparatus according to claim 1, or claim 2, further comprising a temperature maintaining unit for keeping the water supplied into the birth tub within the range of 35°C to 40°C, and a water level controller for controlling the level of the water in the birth tub to be at a predetermined level.

4. The waterbirth apparatus according to any preceding claim, further comprising a chair for a parturient woman, having a downward hole, allowing her to have a baby while sitting on the chair.

5. The waterbirth apparatus according to any preceding claim, wherein a pump for pumping up the water to be supplied to the water purifying unit and a pre-treating unit for pre-treating the water are installed between the water supplying unit and the water purifying unit, and a heating unit for heating to very quickly increase the temperature of the water is installed between the water purifying unit and the birth tube.

6. The waterbirth apparatus according to any preceding claim, wherein the water purifying unit includes a reverse osmotic filter and a carbon filter.

7. The waterbirth apparatus according to any preceding claim, wherein a chair for a parturient woman is detachably installed in the birth tube, and steps are installed inside the outside the birth tub, thus allowing her to easily step in and out of the tub.

8. The use of a waterbirth apparatus according to any preceding claim in the delivery of a child.
